# EUROPÄISCHE PATENTSCHRIFT

(11) **EP 4 404 985 B1**
(45) Veröffentlichungstag und Bekanntmachung des Hinweises auf die Patenterteilung: **09.07.2025**
(21) Anmeldenummer: 22793713.3
(22) Anmeldetag: 22.09.2022
(51) Int. Cl.: A61L 9/20

(54) **LUFTREINIGER ZUR UV-BESTRAHLUNG VON INSBESONDERE MIT KEIMEN ODER INFEKTIONSERREGERN BELADENER STRÖMENDER LUFT**
AIR PURIFIER FOR UV IRRADIATION OF FLOWING AIR, IN PARTICULAR LADEN WITH GERMS OR INFECTIOUS AGENTS
PURIFICATEUR D'AIR POUR L'IRRADIATION UV D'UN AIR EN CIRCULATION EN PARTICULIER CHARGÉ DE GERMES OU D'AGENTS INFECTIEUX

(30) Priorität: 24.09.2021 DE 102021124799
(43) Veröffentlichungstag der Anmeldung: 31.07.2024
(73) Patentinhaber: IST METZ GmbH & Co. KG, 72622 Nürtingen (DE); Virobuster International GmbH, 53639 Königswinter (DE)
(72) Erfinder: STARZMANN, Oliver, 72669 Unterensingen (DE); EBINGER, Klaus, 73249 Wernau (DE); YIGIT, Fehmi, 48599 Gronau (DE)
(74) Vertreter: Pfiz/Gauss Patentanwälte PartmbB
(86) Internationale Anmeldenummer: PCT/EP2022/076356
(87) Internationale Veröffentlichungsnummer: WO 2023/046827

(56) Entgegenhaltungen:
- RU-C1- 2 417 105
- US-B2- 8 816 301
- ANONYMOUS: "Smart Lifting Mobile Uv Disinfection Cart, Hospitals, Restaurants, Hotels,Offices, Living Rooms, Bedrooms", 13 April 2021 (2021-04-13), XP093016206, Retrieved from the Internet <URL:https://web.archive.org/web/20210413021750/https://www.sl-uvsanitizer.com/ultraviolet-sterilizing-lamp-trolley/smart-lifting-mobile-uv-disinfection-cart.html> [retrieved on 20230120]

## Beschreibung

Die Erfindung betrifft einen Luftreiniger zur UV-Bestrahlung von insbesondere mit Keimen oder Infektionserregern beladener strömender Luft.

Aus dem Stand der Technik allgemein bekannte Luftreiniger zeichnen sich dadurch aus, dass eine Lampe in einem Strömungskanal angeordnet ist, wobei durch den Strömungskanal die zu reinigende Luft strömt. Die Lampe sendet dabei Strahlung aus, mit welcher die Keime oder Infektionserreger unschädlich gemacht werden sollen. Der Strömungskanal ist meist mit einem reflektierenden Material ausgekleidet, wobei das von der Lampe ausgesandet Licht reflektiert wird, so dass eine möglichst große Luftmenge bzw. möglichst viele Keime oder Infektionserreger bestrahlt werden können. Je nachdem, wie die Keime oder Infektionserreger durch den Strömungskanal strömen, können bei den aus dem Stand der Technik bekannten Luftreinigern vereinzelt Keime oder Infektionserreger den Luftreiniger passieren, ohne unschädlich gemacht zu werden. Die RU2417105C1 offenbart einen gattungsgemässen Luftreiniger.

Ausgehend hiervon liegt der Erfindung die Aufgabe zugrunde, einen Luftreiniger dahingehend zu verbessern, dass möglichst viele Keime oder Infektionserreger unschädlich gemacht werden.

Zur Lösung dieser Aufgabe wird die in den Patentansprüchen 1 und 14 angegebenen Merkmalskombinationen vorgeschlagen. Vorteilhafte Ausgestaltungen und Weiterbildungen der Erfindung ergeben sich aus den abhängigen Ansprüchen.

Die erfindungsgemäße Lösung umfasst einen Luftreiniger zur UV-Bestrahlung von insbesondere mit Keimen oder Infektionserregern beladener strömender Luft. Der Luftreiniger umfasst einen bevorzugt zylinderförmig ausgebildeten, sich entlang einer Längsachse erstreckenden Außenreflektor. In einer weiteren Ausgestaltung der Erfindung kann der Außenreflektor auch kanalförmig und beispielsweise mit einem rechteckigen, quadratischen oder polygonförmigen Querschnitt ausgeführt sein.

Der Außenreflektor umfasst einen Lufteinlass sowie einen Luftauslass. In den Lufteinlass strömt die zu reinigende Luft in den Luftreiniger ein. Die gereinigte Luft strömt aus dem Luftauslass aus. Der Außenreflektor umfasst an seiner der Luftströmung zugewandten Innenseite ein UV-Strahlung-reflektierendes Material.

Der Luftreiniger umfasst eine UV-Strahlung emittierende, sich entlang einer Längsachse erstreckende stabförmigen Lampe. Zweckmäßigerweise ist die Lampe vom Außenreflektor umgeben. Der Außenreflektor ist vorgesehen, um die von der Lampe ausgesandte Strahlung zu reflektieren. Bevorzugt wird die Strahlung vom Außenreflektor mehrfach, mindestens zweifach, bevorzugt vielfach reflektiert. In einer ganz besonders bevorzugten Ausgestaltung ist die Lampe derart im Außenreflektor angeordnet, dass die vom Außenreflektor reflektierte Strahlung nicht auf die Lampe zurück reflektiert wird, vorteilhafterweise zumindest nicht bei der ersten Reflexion der von der Lampe ausgesandten Strahlung. Insbesondere nach einer mehrfachen Reflexion, beispielsweise zweifachen oder dreifachen Reflexion kann die Strahlung die Lampe treffen, denn die Strahlung kann nach mehrfacher Reflexion insbesondere abgeschwächt sein.

Der Luftreiniger umfasst einen sich entlang einer Längsachse erstreckenden rohr- bzw. stabförmigen Zentralreflektor, der vom Außenreflektor umgeben ist. Der Zentralreflektor umfasst an seiner der Luftströmung und/oder dem Außenreflektor und/oder der Lampe zugewandten Seite ein UV-Strahlung-reflektierendes Material. Vorteilhaft reflektieren der Außenreflektor und der Zentralreflektor die von der Lampe emittierte UV-Strahlung.

Zweckmäßigerweise reflektiert der Zentralreflektor die Strahlung, welche direkt von der Lampe auf den Zentralreflektor trifft. Zweckmäßigerweise reflektiert der Zentralreflektor die Strahlung, welche vom Außenreflektor auf den Zentralreflektor trifft. Mit dem Zusammenspiel aus Lampe, Außenreflektor und Zentralreflektor wird damit erreicht, dass möglichst viel Strahlung auf die Luft und damit auf die Keime oder Infektionserreger trifft, so dass möglichst viel Luft gereinigt wird. Der Zentralreflektor dient insbesondere dazu, die Reflexionswege zu verkürzen. Eine Verkürzung ist vorteilhaft, da die Luft UV-Strahlung absorbiert. Bei verkürzten Reflexionswegen wird eine optimale Unschädlichmachung der Keime oder Infektionserreger erreicht. Darüber hinaus wird durch den schräg gestellten Mittelreflektor eine Rückreflexion im selben Strahlungsbündel vermieden.

Ganz besonders vorteilhaft ist die Längsachse der Lampe schräg zur Längsachse des Außenreflektors ausgerichtet, und/oder vorteilhaft ist die Längsachse der Lampe schräg zur Längsachse des Zentralreflektors ausgerichtet. Mit dem Begriff schräg ist hierbei eine nicht-parallele Ausrichtung gemeint. Unter schräg kann beispielsweise windschief verstanden werden. Unter schräg kann vorteilhaft auch sich schneidend verstanden werden. Vorteilhaft ist unter schräg zu verstehen, dass der Abstand der Längsachse der Lampe und/oder des Zentralreflektors zu einer Unterseite des Außenreflektors am Lufteinlass ungleich ist zum Abstand der Längsachse der Lampe und/oder des Zentralreflektors zur Unterseite des Außenreflektors am Luftauslass. Der Abstand zur Unterseite gemessen soll hierbei lediglich eine Referenzfläche/- seite angeben. Es versteht sich, dass anstelle der Unterseite auch eine andere Stelle des Außenreflektors betrachtet werden kann, beispielsweise eine Seitenfläche, die Oberseite oder dergleichen.

Durch die Schrägstellung wird erreicht, dass sich der Abstand eines Keimes oder Infektionserregers zur Lampe, und/oder zum Zentralreflektor ändert. Bei dieser Änderung tritt meist beim Durchströmen durch den Luftreiniger an irgendeiner Stelle gerade ein solcher Abstand auf, bei welchem die UV-Strahlung eine Intensität aufweist, die zur Abtötung der Keime oder Infektionserreger führt. Je nachdem, in welchem Abstand die Keime oder Infektionserreger in den Luftreiniger einströmen, werden die Keime oder Infektionserreger an einer früheren oder späteren Position im Luftreiniger unschädlich gemacht. Es hat sich gezeigt, dass jedenfalls mehr Keime oder Infektionserreger unschädlich gemacht werden, als bei herkömmlichen Luftreinigern mit nicht-schräg angeordneten Lampen und/oder Zentralreflektoren. Zusammenfassend kann erläutert werden: Ist beim Lufteintritt der Abstand Lampe zu Reflektor groß, so ist die Bestrahlungsstärke kleiner. Beim Luftaustritt ist der Abstand zwischen Lampe und Reflektor klein, dadurch ist die Bestrahlungsstärke höher. Über die Bestrahlungslänge zwischen Luft-Eintritt und Luft-Austritt wird so die Bestrahlungsstärke homogenisiert und maximiert. Dasselbe gilt natürlich für umgekehrte Abstände bei Luftein- und auslass.

Weiter hat sich gezeigt, dass durch die Schrägstellung von Lampe und/oder Zentralreflektor ein Reflexionsbild erreicht wird, bei dem die von der UV-Lampe ausgesandten Strahlen mehrfach reflektiert werden. Durch die Mehrfachreflexion wird sichergestellt, möglichst viele Keime oder Infektionserreger zu treffen. Auch hat sich gezeigt, dass durch die Schrägstellung eine direkte Rückreflexion auf die Lampe vermieden wird. Dadurch wird die Lampe weniger stark aufgeheizt, was die Lebensdauer der Lampe verlängert.

Vorteilhaft ist die Längsachse des Zentralreflektors schräg zur Längsachse des Außenreflektors ausgerichtet. Vorteilhaft sind sowohl die Längsachse der Zentralreflektors als auch die Längsachse des Außenreflektors schrägt zur Längsachse der Lampe angeordnet. Durch eine Schrägstellung von mehreren Längsachsen zueinander kann ein komplexes Reflexionsbild erzeugt werden, wobei hierbei insbesondere möglichst viel UV-Strahlung die Keime oder Infektionserreger trifft, insbesondere in einem Abstand trifft, welcher optimal zur Unschädlichmachung der Keime oder Infektionserreger ist.

Der Zentralreflektor besteht vorteilhaft aus einem, insbesondere gebogenen, Aluminiumblech, das vorteilhaft für eine Reflexion im UVC beschichtet ist. Dieses Aluminiumrohr ist vorteilhaft auf die Halter aufgesteckt. Zweckmäßig ist der Zentralreflektor in eine Ringnut in der Halterung eingefügt.

Vorzugsweise umfasst der Luftreiniger mindestens zwei, bevorzugt drei, besonders bevorzugt mehr als drei Lampen, ganz besonders bevorzugt zehn Lampen. Bevorzugt ist das System modular für unterschiedliche Luftvolumen über den Rohrdurchmesser und die Anzahl der Lampen aufgebaut. Vorteilhaft sind die Lampen symmetrisch verteilt. Es kann zweckmäßig sein, die Lampen asymmetrisch zu verteilen.

Vorteilhaft ist jede der Lampen als eine UV-Strahlung emittierende stabförmige Lampe ausgebildet ist, die sich jeweils entlang einer Längsachse der jeweiligen Lampe erstreckt. Vorzugsweise ist jede der Lampen mindestens teilweise, insbesondere vollständig vom Außenreflektor umgeben. Vorzugsweise reflektieren der Außenreflektor und der Innenreflektor bei eingeschalteten Lampen die von den Lampen emittierte UV-Strahlung. Vorzugsweise ist jede der Lampen mindestens teilweise zwischen Außenreflektor und Zentralreflektor angeordnet ist. Durch die Verwendung von mehreren Lampen kann mehr Strahlungsleistung in den Luftreiniger eingebracht werden. Darüber hinaus hat sich gezeigt, dass die UV-Strahlung, direkt aus der Lampe und/oder ein- oder mehrfach reflektiert von Zentral- und/oder Außenreflektor, aufgrund vorteilhafter Strahlungswege zum Abtöten der Keime oder Infektionserreger führt.

Zweckmäßig umfasst der Luftreiniger eine sternförmige, lufteinlassseitige Halterung. Zweckmäßig verbindet jeweils ein Haltesteg das lufteinlassseitige Ende jeweils einer der Lampen mit dem lufteinlassseitigen Ende des Zentralreflektors mechanisch. Zweckmäßig umfasst der Luftreiniger eine sternförmige, luftauslassseitige Halterung, die insbesondere Haltestege umfasst. Zweckmäßig verbindet jeweils ein Haltesteg das luftauslassseitige Ende jeweils einer der Lampen mit dem luftauslassseitigen Ende des Zentralreflektors mechanisch. Zweckmäßig sind die lufteinlassseitige und die luftauslassseitige Halterung baugleich ausgebildet. Durch die sternförmige Halterung wird eine einfache Positionierung ermöglicht, die insbesondere die Luftströmung möglichst wenig stört. Die sternförmige Halterung ist insbesondere einfach zu montieren. Beispielsweise können die Lampen und der Zentralreflektor zunächst mit der Halterung verbunden werden, und anschließend zusammen mit der Halterung in den Außenreflektor eingesetzt werden. Zweckmäßig ist die sternförmige Halterung mit dem Außenreflektor mechanisch verbunden. Zweckmäßig ist im Zentrum des Sterns der Zentralreflektor befestigt.

Vorteilhaft sind mindestens zwei Haltestege unterschiedlich lang ausgebildet. Vorteilhaft ist die Länge der Haltestege derart gewählt ist, dass der von den Enden der Lampen gebildete geometrische Mittelpunkt von der Längsachse des Zentralreflektors und/oder von der Längsachse des Außenreflektors beabstandet ist. Dadurch kann auf einfache Weise eine Schrägstellung der Lampen relativ zum Zentralreflektor und/oder zum Außenreflektor umgesetzt werden.

Vorzugsweise ist an mindestens einem, insbesondere an allen lufteinlassseitigen und/oder luftauslassseitigen Haltestegen jeweils eine Stromführung zur Versorgung der jeweiligen mit dem Haltesteg verbundenen Lampe mit Strom vorgesehen. Dadurch kann die Stromführung strömungsmechanisch vom Haltesteg verdeckt sein, so dass die Stromführung keinen zusätzlichen Druckverlust im Strömungskanal erzeugt. Bevorzugt sind Öffnungen in der Halterung, insbesondere in den Haltestegen vorgesehen, an welchen bevorzugt die Stromführung, beispielsweise in Form von Kabeln, mechanisch befestigbar ist. Vorzugsweise erfolgt die Stromführung nur am lufteinlassseitigen oder nur am luftauslassseitigen Haltesteg. Dadurch muss nur eine Kabelführung an einer Seite vorgesehen sein, was insbesondere die Montage und auch Wartung vereinfacht. Weiter sind dadurch die Kabel zentral verlegbar und gehen beispielsweise von einem Vorschaltgerät ab.

Zweckmäßig ist die sternförmige, lufteinlassseitige und/oder luftauslassseitige Halterung über mindestens einen Befestigungssteg mit dem Außenreflektor verbunden. Zweckmäßig umfasst der Befestigungssteg eine Stromführung zur Versorgung der Lampen mit Strom.

Vorteilhaft umfasst die Halterung mindestens zwei, bevorzugt drei Befestigungsstege. Dadurch ist eine gute mechanische Stabilität der Halterung bei gleichzeitig niedrigem Druckverlust im Strömungskanal gegeben.

Vorzugsweise sind mindestens zwei Befestigungsstege unterschiedlich lang. Vorzugsweise ist die Länge der Befestigungsstege derart gewählt, dass das Ende des Zentralreflektors, insbesondere das Ende der Längsachse des Zentralreflektors, von der Längsachse des Außenreflektors beabstandet ist. Dadurch kann der Zentralreflektor auf einfache Weise schräg zum Außenreflektor ausgerichtet werden. Es kann zweckmäßig sein, dass entweder nur die lufteinlassseitige Halterung oder nur die luftauslassseitige Halterung Befestigungsstege mit unterschiedlichen Längen aufweist. Besonders vorteilhaft sind sowohl die lufteinlassseitige, als auch die lufteinlassseitige Halterung mit Befestigungsstegen unterschiedlicher Länge vorgesehen. Besonders zweckmäßig sind die Halterungen baugleich ausgebildet.

Zweckmäßig ist die in Richtung der Längsachse des Außenreflektors gemessene Dicke der sternförmigen, lufteinlassseitigen und/oder luftauslassseitigen Halterung kleiner, bevorzugt mindestens zweimal, besonders bevorzugt mindestens fünfmal, ganz besonders bevorzugt mindestens zehnmal kleiner, bevorzugt nicht mehr als 100 mal kleiner, als die orthogonal zur Längsachse des Außenreflektors gemessene Breite der sternförmigen, lufteinlassseitigen und/oder luftauslassseitigen Halterung. Dadurch ist die Halterung vergleichsweise flach. Dadurch wird möglichst wenig Bauraum, insbesondere in Strömungsrichtung der Luftströmung, durch die Halterung verwendet.

Vorteilhaft ist die Lampe mindestens teilweise, insbesondere vollständig, von einem Hüllrohr umgeben. Vorteilhaft ist das Hüllrohr für das von der Lampe emittierte UV-Strahlung durchlässig. Vorteilhaft ist die Lampe optional und insbesondere vollständig, von einem Hüllrohr umgeben. Das Hüllrohr verhindert ein zu starkes Abkühlen der Lampe. Ein zu starkes Abkühlen der Lampe würde zu einem Effizienzverlust führen. Es kann zweckmäßig sein, kein Hüllrohr vorzusehen, insbesondere bei Luftreinigern mit sehr kleinem Luftdurchsatz, beispielsweise mit einem Luftdurchsatz von etwa3000 m³/h oder weniger. Vorzugsweise ist das die Lampe an ihren Enden jeweils von einer Lampenhalterung gehalten, wobei das Hüllrohr an den Lampenhalterungen befestigt ist.

Zweckmäßig verschließt das Hüllrohr die Lampe luftdicht. Damit kann eine direkte Kühlung der Lampe durch Luft, insbesondere durch strömende Luft verhindert werden. Dadurch kann die Lampe besonders günstig ausgelegt werden, so dass die Lampe besonders effizient arbeitet.

Vorteilhaft ist zwischen Hüllrohr und Lampe ein Freiraum vorgesehen. Der Freiraum ermöglicht insbesondere eine Wärmeisolation, so dass das Hüllrohr weniger aufgeheizt wird als ein direkt an der Lampe anliegendes Hüllrohr. Vorzugsweise ist das Hüllrohr aus Kunststoff gefertigt. Es kann zweckmäßig sein, dass das Hüllrohr aus Glas gefertigt ist.

Vorzugsweise umfasst der Zentralreflektor mindestens einen Durchlass zur Kühlung des Zentralreflektors mit Luft. Dadurch kann der Zentralreflektor gekühlt werden, um beispielsweise eine zu hohe Wärmebelastung zu vermeiden. Vorteilhaft umfasst der Durchlass einen Einlass und einen Auslass. Zweckmäßig ist der Auslass so angeordnet, dass der Auslass mit UV-Strahlung der Lampe beaufschlagbar ist. Vorteilhaft ist der Einlass so angeordnet, dass der Einlass mit UV-Strahlung der Lampe beaufschlagbar ist. Dadurch kann die aus dem Durchlass strömende und/oder in den Einlass strömende Luft von Keimen oder Infektionserregern befreit werden. Es kann vorteilhaft sein, dass die in den Durchlass einströmende und/oder aus dem Durchlass ausströmende Luft im Luftreiniger von Keimen oder Infektionserregern befreit ist.

Vorteilhaft ist ein Luftreiniger zur UV-Bestrahlung von insbesondere mit Keimen oder Infektionserregern beladener strömender Luft vorgesehen. Vorteilhaft umfasst der Luftreiniger einen bevorzugt zylinderförmig ausgebildeten, sich entlang einer Längsachse erstreckenden Außenreflektor, der einen Lufteinlass sowie einen Luftauslass umfasst, wobei der Außenreflektor an seiner der Luftströmung zugewandten Innenseite ein UV-Strahlung-reflektierendes Material umfasst. Vorteilhaft umfasst der Luftreiniger einen sich entlang einer Längsachse erstreckenden stabförmigen Zentralreflektor, der vom Außenreflektor umgeben ist, wobei der Zentralreflektor an seiner der Luftströmung und/oder dem Außenreflektor und/oder der Lampe zugewandten Seite ein UV-Strahlung- reflektierendes Material umfasst. Vorteilhaft umfasst der Luftreiniger eine UV-Strahlung emittierende, sich entlang einer Längsachse erstreckende stabförmige Lampe, wobei die Lampe zwischen Außenreflektor und Zentralreflektor angeordnet ist. Vorteilhaft reflektiert der Außenreflektor und der Zentralreflektor das von der Lampe emittierte UV-Strahlung. Vorteilhaft ist die Längsachse der Außenreflektors schräg zur Längsachse des Zentralreflektors ausgerichtet.

Ein weiterer Erfindungsaspekt liegt in einem Luftreiniger zur UV-Bestrahlung von insbesondere mit Keimen oder Infektionserregern beladener strömender Luft, mit einem bevorzugt zylinderförmig ausgebildeten Außenreflektor, der einen Lufteinlass sowie einen Luftauslass umfasst, wobei der Außenreflektor an seiner der Luftströmung zugewandten Innenseite ein UV-Strahlung-reflektierendes Material umfasst, mit einem Zentralreflektor, der vom Außenreflektor umgeben ist, wobei der Zentralreflektor an seiner der Luftströmung zugewandten Außenseite ein UV-Strahlung reflektierendes Material umfasst, und mit mehreren, bevorzugt mindestens drei stabförmigen Lampen, die eine UV-Strahlung emittieren, wobei die Lampen zwischen Außenreflektor und Zentralreflektor angeordnet sind, und wobei der Außenreflektor und der Zentralreflektor die von der Lampe emittierte UV-Strahlung reflektieren.

Vorteilhaft sind die Lampen in einem Ringraum bzw. Ringbereich um den Zentralreflektor verteilt angeordnet, insbesondere in Blickrichtung entlang der Längsachse des Außenreflektors oder des Zentralreflektors. Bevorzugt weißen jeweils zwei benachbarte Lampen den gleichen Abstand zueinander auf, insbesondere gemessen von einem Ende der einen Lampe zu einem Ende der benachbarten Lampe.

Im Folgenden wird die Erfindung anhand der in der Zeichnung in schematischer Weise dargestellten Ausführungsbeispiele näher erläutert. Es zeigen:
- Fig. 1: eine Vorderansicht eines Luftreinigers,
- Fig. 2: eine Hinteransicht des Luftreinigers,
- Fig. 3: eine schematische Darstellung von Reflexionen von Lichtstrahlen bei einer Schrägstellung von Lampe und/oder Zentralreflektor,
- Fig. 4: eine schematische Darstellung von Reflexionen von Lichtstrahlen bei einer nicht-schräggestellten Lampe nach dem Stand der Technik,
- Fig. 5: eine graphische Darstellung von Intensitätsverläufen von schräg und nicht-schräg angeordneten Lampen,
- Fig. 6: eine Schnittansicht der Lampe mit einem Hüllrohr,
- Fig. 7: eine vergrößerte Detailansicht eines Endes der in Fig. 6 gezeigten Lampe mit Hüllrohr,
- Fig. 8: eine vergrößerte Detailansicht eines anderen Endes der in Fig. 6 gezeigten Lampe mit Hüllrohr,
- Fig. 9: eine schematische Darstellung in einer teilgeschnittenen Ansicht einer Kabelführung zu der Lampe,
- Fig. 10: eine vergrößerte Detailansicht eines Endes der in Fig. 6 gezeigten Lampe mit Hüllrohr und mit einem Stecker,
- Fig. 11: eine vergrößerte Detailansicht eines anderen Endes der in Fig. 6 gezeigten Lampe mit Hüllrohr und mit einem Stecker.

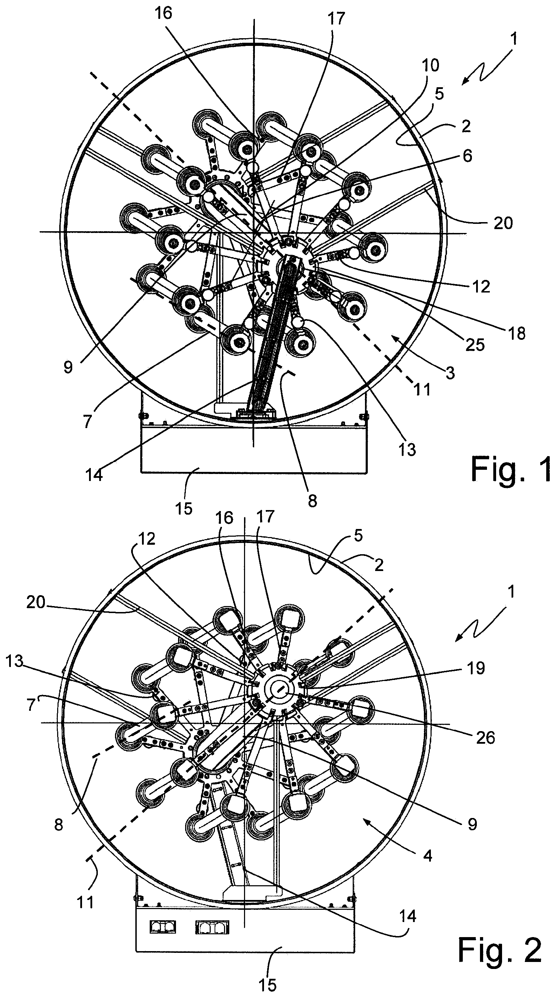

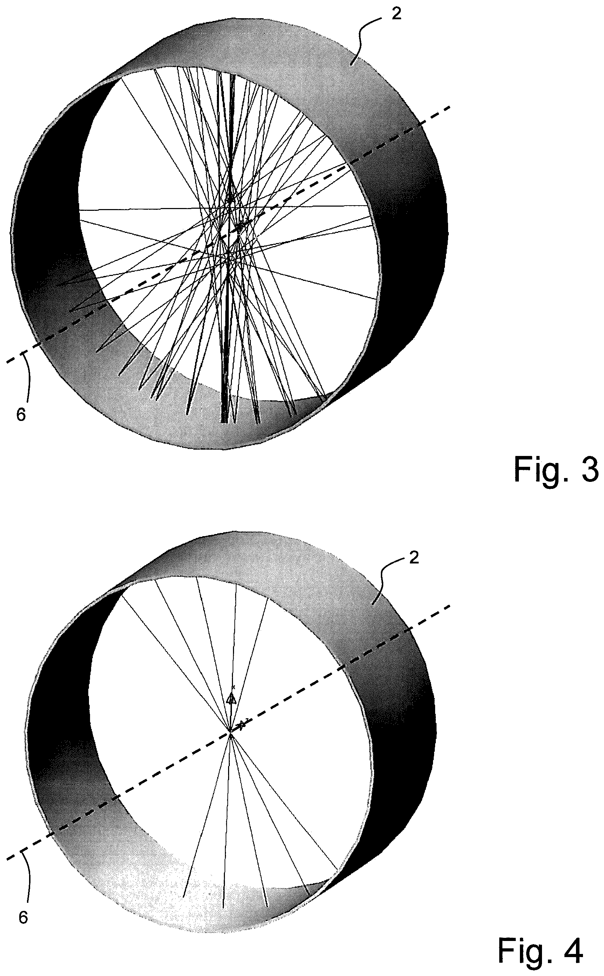

Fig. 1 und 2 zeigen einen Luftreiniger 1, welcher zur UV-Bestrahlung von mit Keimen oder Infektionserregern beladener strömender Luft vorgesehen ist. Hierbei können etwa bis etwa 6000 m³/h Luft gereinigt werden. Der Luftreiniger 1 umfasst einen im Ausführungsbeispiel kreiszylindrisch ausgebildeten Außenreflektor 2. Der Außenreflektor 2 weist einen Innendurchmesser von etwa 400 mm bis etwa 500 mm auf, wobei 500 mm bevorzugt sind. In weiteren Ausführungsbeispielen kann der Außenreflektor 2 kanalförmig, beispielsweise mit einem vier- oder mehreckigen Querschnitt ausgebildet sein. Der Außenreflektor 2 erstreckt sich entlang einer Längsachse 6. In den Fig. 1 und 2 geht die Längsachse 6 des Außenreflektors 2 in die Blattebene hinein bzw. führt aus der Blattebene heraus. Die Längsachse 6 des Außenreflektors 2 ist gut in den Fig. 3 und 4 zu sehen. Der Außenreflektor 2 bildet einen Strömungskanal für die strömende Luft. Im Allgemeinen strömt die Luft etwa parallel zur Längsachse 6 des Außenreflektors 2. Hierzu umfasst der Außenreflektor 2 einen Lufteinlass 3 und einen Luftauslass 4.

Fig. 1 zeigt den Luftreiniger 1 in einer Vorderansicht, wobei der Lufteinlass 3 zu sehen ist. Die Luft strömt in Fig. 1 in die Blattebene hinein. Fig. 2 zeigt den Luftreiniger in einer Hintenansicht, wobei der Luftauslass 4 zu sehen ist. Die Luft strömt in Fig. 2 aus der Blattebene heraus. Zweckmäßig sind Lufteinlass 3 und Luftauslass 4 nicht verengt. Dadurch kann der Luftreiniger 1 einfach in ein bestehendes Rohrsystem eingesetzt werden.

Der in Fig. 1 und 2 gezeigte Außenreflektor 2 umfasst ein UV-Strahlung reflektierendes Material 5. Das UV-Strahlung reflektierende Material 5 ist an der Seite des Außenreflektors 2 angebracht, welche der Luftströmung zugewandt ist. Im Ausführungsbeispiel eines zylinderförmig ausgebildeten Außenreflektors 2 ist das UV-Strahlung reflektierende Material 5 an der Innenseite des Außenreflektors 2 angebracht.

Der Luftreiniger 1 umfasst im Ausführungsbeispiel zehn Lampen 7. Es kann zweckmäßig sein, dass der Luftreiniger 1 in einem weiteren Ausführungsbeispiel mindestens eine Lampe 7, bevorzugt mindestens zwei Lampen 7, besonders bevorzugt mindestens vier Lampen 7 umfasst. Es kann zweckmäßig sein, dass der Luftreiniger in einem weiteren Ausführungsbeispiel höchstens fünfzig Lampen 7, bevorzugt höchstens zwanzig Lampen 7, besonders bevorzugt höchstens sechzehn Lampen 7 umfasst. Die Lampen 7 sind im Ausführungsbeispiel baugleich ausgebildet. Im Folgenden wird daher nur eine der Lampen 7 beschrieben. Das für die eine Lampe 7 Gesagte gilt folgerichtig für eine oder mehrere der, insbesondere alle, Lampen 7.

Die Lampe 7 weist eine Stabform auf. Die stabförmige Lampe 7 erstreckt sich demnach in im Wesentlichen entlang einer Längsachse 8. Der Querschnitt der Lampe 7 ist im Ausführungsbeispiel im Wesentlichen und größtenteils - insbesondere abgesehen von den Enden der Lampe 7 - etwa kreisförmig. Die Lampe 7 emittiert im Betrieb eine UV-Strahlung. Die UV-Strahlung ist bevorzugt derart, dass die UV-Strahlung Keime oder Infektionserreger unschädlich machen kann. Hierbei ist die UV-Strahlung sowohl in der Wellenlänge als auch in der Intensität angepasst.

Im Ausführungsbeispiel handelt es sich um Niederdrucklampen, mit einer nutzbringenden Strahlung im Bereich von 254 Nanometer. Diese sind energetisch günstiger und verbrauchsoptimiert im Vergleich zu Quecksilber-Mitteldrucklampen. In einem weiteren Ausführungsbeispiel können Niederdrucklampen, Mitteldrucklampen, und/oder Excimerlampen als mögliche Strahlungsquellen vorgesehen sein. In einem weitere Ausführungsbeispiel kann die Lampe mit einer stabförmigen Anordnungen von LEDs gebildet sein. Bevorzugt arbeiten die LEDs im Wellenlängenbereich von 254 Nanometer, insbesondere UVC. Bevorzugt handelt es sich um UVC-LEDs.

Wie gut in den Fig. 1 und 2 zu erkennen, ist die Lampe 7, bzw. sind sämtliche Lampen 7, vom Außenreflektor 2 umgeben. Die von der Lampe 7 emittierte Strahlung wird hierbei vom Außenreflektor 2 meist mindestens einmal, oftmals mehrfach reflektiert. Das Reflexionsbild der UV-Strahlen durch den Außenreflektor 2 soll nachstehend anhand der Fig. 3 und 4 näher erläutert werden.

Der in Fig. 1 und 2 gezeigte Luftreiniger 1 umfasst einen Zentralreflektor 9. Der Zentralreflektor 9 ist von Außenreflektor 2 umgeben. Wie gut in Fig. 1 und 2 zu sehen ist, sind die Lampen 7 zwischen dem Zentralreflektor 9 sowie dem Außenreflektor 2 angeordnet. Der Zentralreflektor 9 soll im Wesentlichen die von den Lampen 7 in Richtung auf den Zentralreflektor 9 emittierten UV-Strahlen reflektieren, bevorzugt in Richtung auf den Außenreflektor 2. Der Zentralreflektor 9 soll darüber hinaus die von dem Außenreflektor 2 in Richtung auf den Zentralreflektor 9 reflektierten UV-Strahlen abermals reflektieren, bevorzugt in Richtung auf den Außenreflektor 2. Hierzu umfasst der Zentralreflektor 9 an seiner der Luftströmung und/oder dem Außenreflektor 2 und/oder der Lampe 7 zugewandten Seite ein UV-Strahlung reflektierendes Material 10.

Bevorzugt sind der Zentralreflektor 9 und der Außenreflektor 2 derart angeordnet, dass möglichst viele UV-Strahlen reflektiert werden können, und insbesondere dass möglichst wenig UV-Strahlen zurück auf die Lampen 7 reflektiert werden. Durch die einfache und/oder mehrfache Reflexion der UV-Strahlen kann gewährleistet werden, dass möglichst viele in der Luft strömende Keime oder Infektionserreger mit UV-Strahlung beaufschlagt werden. Ein Keim oder Infektionserreger kann dabei auch mehrfach von der UV-Strahlung beaufschlagt werden.

Wie gut in den Figuren 1 und 2 zu sehen ist, ist der Zentralreflektor 9 rohrförmig ausgebildet mit einem insbesondere kreisförmigen Querschnitt. Der Zentralreflektor erstreckt sich entlang einer Längsachse 11.

Im Luftreiniger 1 nach dem Ausführungsbeispiel ist die die Längsachse 8 der Lampe 7, bzw. sind die Längsachsen 8 der Lampen 7, schräg zur Längsachse 6 des Außenreflektors 2 ausgerichtet. Im Luftreiniger 1 nach dem Ausführungsbeispiel ist die Längsachse 8 der Lampe 7 schräg zur Längsachse 11 des Zentralreflektors 9 ausgerichtet ist. Im Luftreiniger 1 nach dem Ausführungsbeispiel ist die Längsachse 11 des Zentralreflektors 9 schräg zur Längsachse 6 des Außenreflektors 2 ausgerichtet.

Die schräge Anordnung der Längsachsen 6, 8, 11 relativ zueinander ist gut in den Fig. 1 und 2 zu sehen. Die Längsachsen 6, 8, 11 sind windschief und/oder können sich schneiden. Die Längsachsen 6, 8, 11 sind nicht parallel zueinander. Der Winkel der Längsachsen 6, 8, 11 relativ zueinander beträgt mindestens etwa 1°, bevorzugt mindestens etwa 2°, besonders bevorzugt mindestens etwa 3° und höchstens etwa 10°, bevorzugt höchstens etwa 8°, besonders bevorzugt höchstens etwa 6°. In einem weiteren Ausführungsbeispiel beträgt der Winkel der Längsachsen 6, 8, 11 relativ zueinander bevorzugt höchstens etwa 25°. Der Winkel kann beispielsweise von der Größe des Querschnitts abhängen, wobei insbesondere ein größerer Querschnitt zu einem größeren Winkel führen kann. Der Winkel kann von der Länge der Lampe 7 abhängen, wobei insbesondere eine kürzere Länge zu einem größeren Winkel führen kann.

Die Funktion der schrägen Anordnung der Längsachsen 6, 8, 11 kann gut anhand der Fig. 3 und 4 erklärt werden. Fig. 3 und 4 zeigen den Außenreflektor 2 mit UV-Strahlen, insbesondere einem Strahlenbündel von vier Strahlen, die im Außenreflektor 2 reflektiert werden. In Fig. 4 ist hierbei die Längsachse 8 der Lampe 7 identisch zur Längsachse 6 des Außenreflektors 2 ausgerichtet. Die UV-Strahlen treffen sich hierbei auf der Längsachse 6, 8. Wird nun ein Keim oder Infektionserreger vergleichsweise nahe der Längsachse 6, 8 transportiert, so wird der Keim oder Infektionserreger aufgrund der Nähe zur Lampe 7 mit einer vergleichsweise hohen UV-Strahlung belastet. Fliegt nun ein Keim oder Infektionserreger vergleichsweise weiter entfernt von der Längsachse 6, 8, so wird der Keim oder Infektionserreger aufgrund der Entfernung von der Lampe 7 mit einer vergleichsweise niedrigen UV-Strahlung belastet. Die UV-Bestrahlung der Keime oder Infektionserreger ist hierbei stark abhängig davon, wie nah oder entfernt von der Lampe 7 der Keim oder Infektionserreger fliegt.

In Fig. 3 ist die Längsachse 8 der Lampe 7 schräg zur Längsachse 6 des Außenreflektors 2 ausgerichtet. Die UV-Strahlen treffen sich hierbei meist nicht mehr auf der Längsachse 6, 8, sondern werden mehrfach reflektiert. Die Reflexionen können insbesondere auch an den Lampen 7 vorbeiführen. Hierbei wird vergleichsweise viel UV-Strahlung innerhalb des Luftreinigers 1 gehalten. Durch die Schrägstellung der Lampen 7, bzw. analog des Zentralreflektors 9, wird außerdem erreicht, dass die Lampen 7 schrägt im Strömungskanal des Außenreflektors 2 stehen. Das hat zur Folge, dass beispielsweise das einlassseitige Ende der Lampe 7 näher am Außenreflektor 2 liegt, als das auslassseitige Ende der Lampe 7, siehe auch Fig. 1 und 2. Entsprechendes gilt für den Zentralreflektor 9. Fliegt nun ein Keim oder Infektionserreger in einer beliebigen Distanz von der Längsachse 6 des Außenreflektors, so wird der Keim oder Infektionserreger aufgrund der Schrägstellung der Lampe 7 während des Flugs durch den Luftreiniger mit unterschiedlich hoher UV-Strahlung belastet, da der Keim oder Infektionserreger an gewissen Stellen nahe an der Lampe 7 ist und an anderen Stellen weiter entfernt von der Lampe 7 ist. Dabei stellt sich mit hoher Wahrscheinlichkeit eine Stelle ein, die genau so weit von der Lampe 7 entfernt ist, dass der Keim oder Infektionserreger unschädlich gemacht wird. Darüber hinaus erfährt der Keim oder Infektionserreger aufgrund der mehrfachen, komplexen Reflexionen, die beispielhaft in Fig. 3 gezeigt sind, eine UV-Bestrahlung von verschiedenen Seiten. Auffällig ist außerdem die sehr gleichmäßige Verteilung der Strahlung über die Fläche.

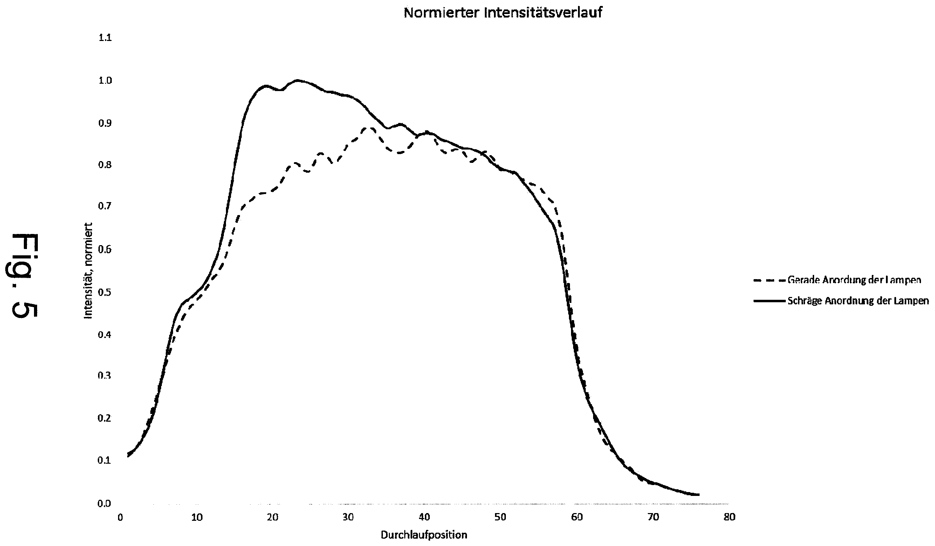

Es hat sich gezeigt, dass eine schräge Anordnung von Lampen 7, Außenreflektor 2 und/oder Zentralreflektor 9 relativ zueinander die Keime oder Infektionserreger mit einer höheren Intensität beaufschlagt, wie gut in Fig. 5 zu sehen ist. Fig. 5 zeigt ein Diagramm, in welchem der Intensitätsverlauf in Abhängigkeit der Durchlaufposition dargestellt ist. Verglichen wird eine Anordnung mit schräggestellten Lampen 7 mit einer Anordnung mit geraden, also parallel ausgerichteten Lampen 7. Wie gut zu sehen ist, ist die Intensität bei den schräggestellten Lampen 7 nahezu über gesamten Durchlauf höher als die Intensität bei den geraden, also parallelen Lampen 7 - mit nur wenigen Ausnahmen.

Der in Fig. 1 und 2 gezeigte Luftreiniger 1 umfasst eine sternförmige lufteinlassseitige Halterung 12. Die sternförmige lufteinlassseitige Halterung 12 umfasst je einen Haltesteg 13. An jedem Haltesteg 13 ist ein Ende einer Lampe 7 befestigt. Die sternförmige lufteinlassseitige Halterung 12 ist mit einem Fuß 14 am Außenreflektor 2 und/oder an einem Sockel 15 befestigt. Damit verbindet jeweils ein Haltesteg 13 das lufteinlassseitige Ende jeweils einer der Lampen 7 mit dem lufteinlassseitigen Ende des Zentralreflektors 2 mechanisch. Zweckmäßig sind Öffnungen oder Befestigungsvorrichtungen am Fuß 14 vorgesehen, so dass Kabel am Fuß 14 befestigbar sind.

Die Haltestege 13 erstrecken sich von einer zentralen lufteinlassseitigen Aufnahme 18, die insbesondere Bestandteil der sternförmigen lufteinlassseitigen Halterung 12 ist. Die zentrale lufteinlassseitige Aufnahme 18 ist im Ausführungsbeispiel etwa kreisförmig ausgebildet. An der zentralen lufteinlassseitigen Aufnahme 18 ist ein Ende des Zentralreflektors 9 befestigt.

Der Luftreiniger 1 umfasst eine sternförmige, luftauslassseitige Halterung 16. Die sternförmige luftauslassseitige Halterung 16 umfasst je einen Haltesteg 17. An jedem Haltesteg 17 ist ein Ende einer Lampe 7 befestigt. Die sternförmige luftauslassseitige Halterung 16 ist über die Lampen 7 und/oder über den Zentralreflektor 9 mit der sternförmigen lufteinlassseitigen Halterung 12 und damit über den Fuß 14 verbunden.

Die Haltestege 17 erstrecken sich von einer zentralen luftauslassseitigen Aufnahme 19, die insbesondere Bestandteil der sternförmigen luftauslassseitigen Halterung 16 ist. Die zentrale luftauslassseitige Aufnahme 19 ist im Ausführungsbeispiel etwa kreisförmig ausgebildet. An der zentralen luftauslassseitigen Aufnahme 19 ist ein Ende des Zentralreflektors 9 befestigt.

Im Ausführungsbeispiel sind die lufteinlass- und luftauslassseitigen Halterungen 12, 16 baugleich ausgeführt. Damit eine Schrägstellung der Lampen 7 konstruktiv einfach herstellbar ist, ist vorgesehen, dass mindestens zwei Haltestege 13 der sternförmigen lufteinlassseitigen Halterung 12 bzw. mindestens zwei Haltestege 17 der sternförmigen lufteinlassseitigen Halterung 16 unterschiedlich lang sind. Bevorzugt sind die jeweils benachbarten Haltestege 13, 17 unterschiedlich lang ausgebildet. Es kann besonders zweckmäßig sein, dass die Länge der Haltestege 13, 17 derart gewählt ist, dass der von den Enden der Lampen 7 gebildete geometrische Mittelpunkt von der Längsachse 11 des Zentralreflektors 9 und/oder von der Längsachse 6 des Außenreflektors 2 beabstandet ist.

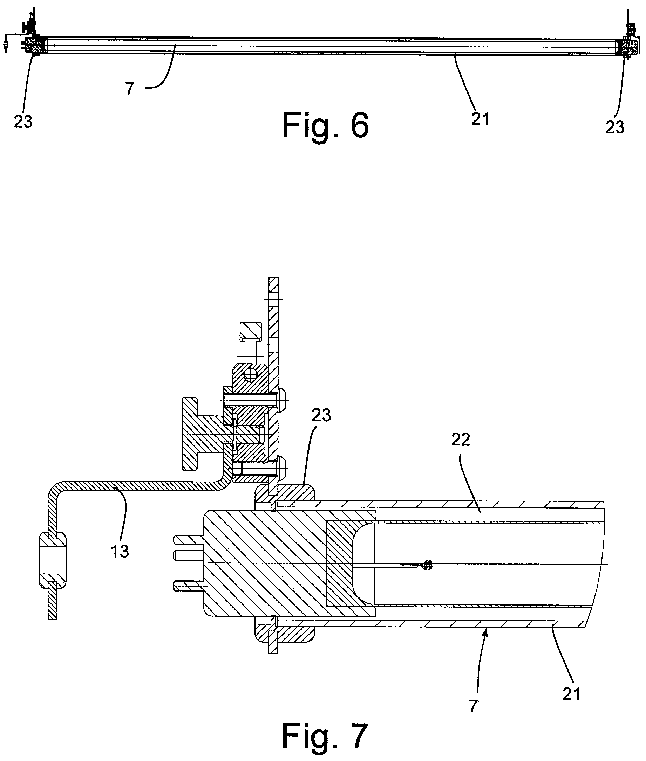

In einem weiteren Ausführungsbeispiel können die lufteinlass- und luftauslassseitigen Halterungen 12, 16 unterschiedlich ausgebildet sein, beispielsweise bezüglich einer Kabelführung, die nachfolgend anhand der Fig. 6 bis 9 beschrieben wird.

Der Zentralreflektor 9 sitzt zwischen der Mittelachse und einer äußeren Ringreihe von Löchern der Halterung 12, 16. Die Mitte der Halterung 12, 16 ist im Ausführungsbeispiel geschlossen, während über die Bohrungen der Halterung 12, 16 Luft zur außenseitigen Kühlung des Zentralreflektors 9 strömen kann. Diese Luft wird ebenfalls bestrahlt.

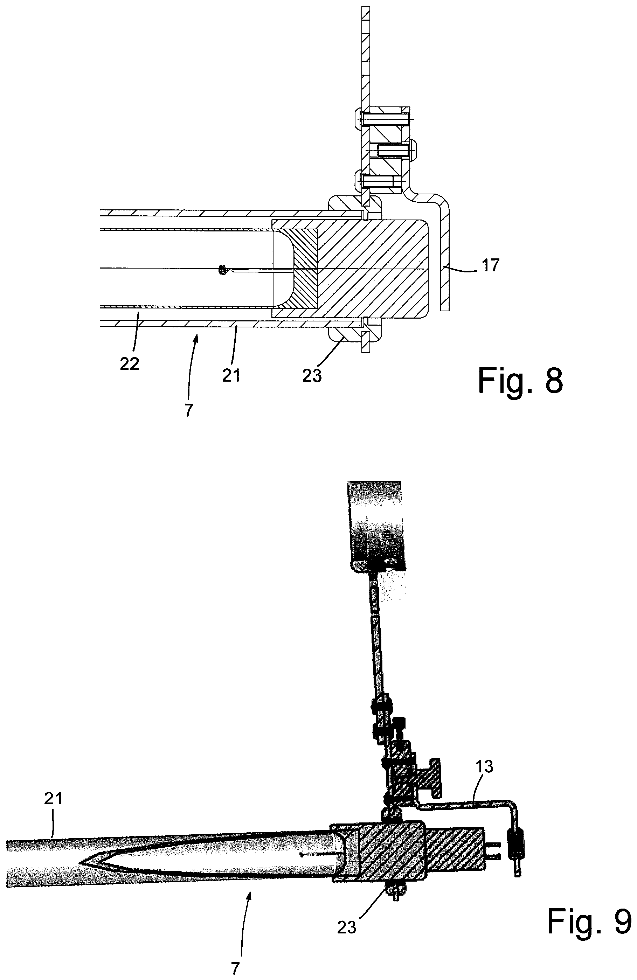

Im Ausführungsbeispiel ist die Lampe 7 an nur einem Ende mit einer Stromzuführung versehen. In der in Fig. 6 gezeigten Darstellung der Lampe 7 handelt es sich um das linke Ende der Lampe 7. Das linke Ende der Lampe 7 ist vergrößert in Fig. 7 und 9 dargestellt, das rechte Ende der Lampe 7 ist vergrößert in Fig. 8 dargestellt. Aufgrund der nur einseitigen Kabelzuführung zur Lampe 7 muss nur an jeweiligen lufteinlassseitigen Haltesteg 13 oder luftauslassseitigen Haltesteg 17 jeweils eine Stromführung, also beispielsweise ein Stromkabel, zur Stromversorgung der jeweiligen mit dem Haltesteg 13, 17 verbundenen Lampe 7 vorgesehen sein. Im Ausführungsbeispiel ist zur Stromführung die lufteinlassseitige Halterung 12 und sind damit die lufteinlassseitigen Haltestege 13 vorgesehen. In einem weiteren Ausführungsbeispiel können die luftauslassseitigen Haltestege 17 zur Stromführung vorgesehen sein. In einem weiteren Ausführungsbeispiel können die lufteinlassseitigen und die luftauslassseitigen Haltestege 13, 17 vorgesehen sein, wobei insbesondere die Lampe an einem Ende oder an beiden Enden mit Strom versorgbar ist.

Die in Fig. 1 und 2 gezeigte sternförmige, lufteinlassseitige Halterung 12 ist über mindestens einen Befestigungssteg 20 mit dem Außenreflektor 2 verbunden. In einem weiteren Ausführungsbeispiel kann die sternförmige, luftauslassseitige Halterung 16 über mindestens einen Befestigungssteg 20 mit dem Außenreflektor 2 verbunden sein. Im Ausführungsbeispiel umfasst die Halterung 12, 16 über drei Befestigungsstege 20. Die drei Befestigungsstege 20 sind in einem gleichen Winkel voneinander beabstandet, also etwa 120°. Es kann zweckmäßig mehr als drei Befestigungsstege 20 vorzusehen.

Vorteilhaft sind alle Befestigungsstege 20 etwa gleich dimensioniert, insbesondere in deren Quererstreckung. Es kann aber auch vorteilhaft sein, dass mindestens einer der Befestigungsstege 20 anders dimensioniert ist. Im in Fig. 1 und 2 gezeigten Ausführungsbeispiel ist einer der Befestigungsstege 20 als Fuß 14 ausgebildet. Der Fuß 14 ist hierbei etwas massiver konstruiert als die übrigen Befestigungsstege 20. Dadurch sind am Fuß ein oder mehrere Stromzuführungen, insbesondere Kabel, befestigbar.

Im Ausführungsbeispiel erfolgt die Stromzuführung über je ein Kabel pro Lampe 7, wobei von jeder Lampe 7 das Kabel über den Haltesteg 13 zum Sterninneren der sternförmigen lufteinlassseitigen Halterung 12 geführt ist. Im Sterninneren der sternförmigen lufteinlassseitigen Halterung 12 treffen sich die zehn Kabel der zehn Lampen 10. Diese Kabel sind vom Sterninneren der sternförmigen lufteinlassseitigen Halterung 12 über den Fuß 14 nach außen geführt. Zweckmäßig sind Öffnungen oder Befestigungsvorrichtungen an den Halterungen 12, 16, insbesondere an den Haltestegen 13, 17, vorgesehen, so dass die Kabel an den Halterungen 12, 16, insbesondere an den Haltestegen 13, 17, befestigbar sind.

Jede der Lampen 7 besitzt folgerichtig ein eigenes Kabel zu einem in den Figuren nicht gezeigten Vorschaltgerät, um die Lampe 7 nur von einer Seite zu Bestromen und gesondert zu zünden. Das oder die Vorschaltgeräte sind in einem ausgelagerten Servicebereich, der im Sockel 15 angeordnet sein kann, angeordnet. Der Servicebereich befindet sich, insbesondere direkt, unterhalb des Außenreflektors 2.

Vorteilhaft sind mindestens zwei Befestigungsstege 20, im Ausführungsbeispiel alle drei Befestigungsstege 20 pro Halterung 12, 16 unterschiedlich lang ausgebildet. Die Länge der Befestigungsstege 20 ist hierbei so gewählt, dass das Ende des Zentralreflektors 2, insbesondere das Ende der Längsachse 11 des Zentralreflektors 9 von der Längsachse 6 des Außenreflektors 2 beabstandet ist. Dadurch ist gewährleistet, dass der Zentralreflektor 9 am Lufteinlass 3 und/oder Luftauslass 4 außermittig vom Außenreflektor 2 angeordnet ist, wodurch die Schrägstellung des Zentralreflektors 9 relativ zum Außenreflektor umsetzbar ist.

Die in Richtung der Längsachse 6 des Außenreflektors 2 gemessene Dicke der sternförmigen, lufteinlassseitigen und/oder luftauslassseitigen Halterung 12, 16 ist kleiner als die orthogonal zur Längsachse 6 des Außenreflektors 2 gemessene Breite der sternförmigen, lufteinlassseitigen und/oder luftauslassseitigen Halterung 12, 16. Die Halterung 12, 16 ist damit flach. Bevorzugt ist die Halterung 12, 16 aus einem Metall, insbesondere aus einem Metallblech gefertigt. Es kann aber auch zweckmäßig sein, ein anderes Material, wie beispielsweise einen Kunststoff einzusetzen. Durch die flache Bauweise wird die Luftströmung wenig beeinflusst. Außerdem wird durch die flache Bauweise der verfügbare Raum für die Lampen 7 und/oder den Zentralreflektor 9 vergrößert.

In den Fig. 6 bis 9 ist die Lampe 7 bzw. sind Details der Lampe 7 dargestellt. Die Lampe 7 ist von einem Hüllrohr 21 umgeben. Das Hüllrohr 21 ist für die von der Lampe 7 ausgestrahlte UV-Strahlung durchlässig und besitzt insbesondere eine Transmission von fast etwa 100 % im relevanten Strahlungsbereich. Zwischen Hüllrohr 21 und Lampe 7 ist ein Freiraum 22, beispielsweise in Form eines Spaltes, vorgesehen. Der Freiraum 22 sorgt für einen Abstand zwischen Hüllrohr 21 und Lampe 7. Es kann zweckmäßig sein, dass der Freiraum 22 mit einem Medium, beispielsweise Luft, einem Gas, oder dergleichen gefüllt ist, beispielsweise als thermischer Isolator. Zweckmäßig verschließt das Hüllrohr 21 die Lampe 7 luftdicht, insbesondere von frischer Luft aus der Durchströmung durch den Außenreflektor 2. Das Hüllrohr 21 schützt die Lampe 7 insbesondere vor zu starker Abkühlung durch die Luftströmung. Mit dem Hüllrohr 21 kann der Luftreiniger 1 einen Luftdurchsatz von etwa 3000 m³/h bis etwa 9000 m³/h, bevorzugt bis etwa 6000 m³/h umsetzen. Ohne Hüllrohr 21 hingegen sind Luftdurchsätze von höchstens etwa 3000 m³/h möglich. Im Ausführungsbeispiel beträgt der Außendurchmesser des Hüllrohrs etwa 25 mm und der Außendurchmesser der Lampe etwa 19 mm. Damit können die thermischen Bedingungen an der Lampe stabil gehalten werden.

Besonders zweckmäßig umgibt das Hüllrohr 21 einen Bereich, der auch für die Rückführung der Anschlusskabel nutzbar ist. Zweckmäßig sind Dichtungen an beiden Seiten am Lampensockel vorgesehen.

Die Lampe 7 umfasst an ihren Enden jeweils eine Lampenhalterung 23. Die Lampe 7 ist an der Lampenhalterung 23 befestigt. Im Ausführungsbeispiel ist auch das Hüllrohr 21 an der Lampenhalterung 23 befestigt. Die Lampenhalterung 23 ist in der sternförmigen, lufteinlassseitigen und/oder luftauslassseitigen Halterung 12, 16, insbesondere an deren Haltestegen 13, 17.

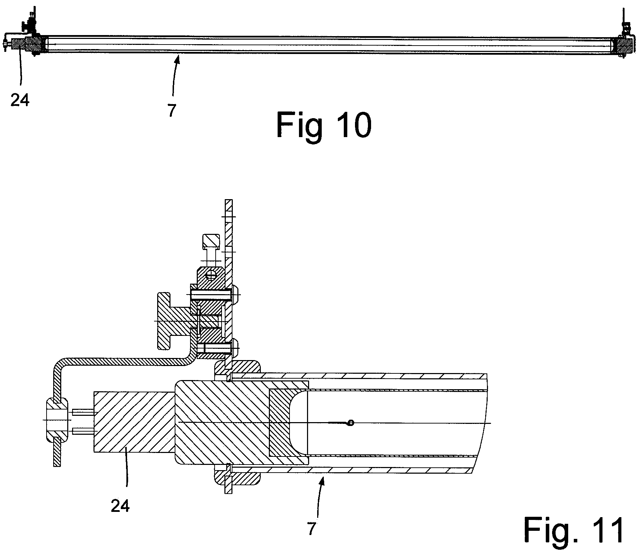

Die in den Fig. 10 und 11 gezeigte Lampe 7 entspricht weitgehend der in Fig. 6 und 7 dargestellten Lampe 7 mit dem Unterschied, dass bei der in Fig. 10 und 11 gezeigten Lampe 7 an einem stirnseitig Ende der Lampe 7 ein elektrischer Stecker 24 angeordnet ist. Der elektrische Stecker 24 ist bevorzugt stirnseitig auf die Lampe 7 aufgesteckt. Damit kann der Stecker 24 einfach an- und abgesteckt werden, was beispielsweise ein einfaches Austauschen der Lampe 7 ermöglicht.

Zweckmäßig sind die Lampen 7, insbesondere das Lampensystem umfassend Lampen und Halterungen, in einen bestehenden Lüftungskanal einschiebbar. Zweckmäßig ist der Luftreiniger 1 in einen bestehenden Lüftungskanal einsetzbar.

Im Ausführungsbeispiel umfasst der Zentralreflektor 9 mindestens einen, in den Figuren 1 und 2 gezeigten Durchlass 25, 26 zur Kühlung des Zentralreflektors 9 mit Luft. Die zu kühlende Luft entstammt insbesondere aus der durch den Außenreflektor 2 strömenden Luft. Im Ausführungsbeispiel sind zwei Durchlässe 25, 26 vorgesehen. Ein erster Durchlass 25 ist zweckmäßig stromab des Lufteinlasses 3 angeordnet. Ein zweiter Durchlass 26 ist zweckmäßig stromauf des Luftauslasses 5 angeordnet. Damit kann Luft in den Zentralreflektor in den ersten Durchlass 25 einströmen und aus dem zweiten Durchlass 26 ausströmen. Bei nur einem Durchlass 25, was in einem weiteren Ausführungsbeispiel möglich ist, strömt die Luft durch den Durchlass 25 ein und auch wieder aus dem Durchlass 25 aus.

Zweckmäßig ist der Durchlass 25, 26 so angeordnet, dass die einströmende Luft bereits durch UV-Strahlen gereinigt wurde, und/oder dass die ausströmende Luft durch UV-Strahlen gereinigt wird, bevor die Luft aus dem Luftauslass 4 aus dem Luftreiniger 1 ausströmt. So kann sichergestellt werden, dass sämtliche Luft, insbesondere auch die durch den Zentralreflektor strömende Luft, von Keimen oder Infektionserregern gereinigt ist.

## Patentansprüche

1. Luftreiniger zur UV-Bestrahlung von insbesondere mit Keimen oder Infektionserregern beladener strömender Luft, mit
- einem bevorzugt zylinderförmig ausgebildeten, sich entlang einer Längsachse (6) erstreckenden Außenreflektor (2), der einen Lufteinlass (3) sowie einen Luftauslass (4) umfasst, wobei der Außenreflektor (2) an seiner der Luftströmung zugewandten Innenseite ein UV-Strahlung-reflektierendes Material (5) umfasst,
- einer UV-Strahlung emittierenden, sich entlang einer Längsachse (8) erstreckenden stabförmigen Lampe (7), wobei die Lampe (7) vom Außenreflektor (2) umgeben ist, wobei der Außenreflektor (2) die von der Lampe (7) emittierte UV-Strahlung reflektiert,
**gekennzeichnet durch**
- einen sich entlang einer Längsachse (11) erstreckenden Zentralreflektor (9), der vom Außenreflektor (2) umgeben ist, wobei der Zentralreflektor (9) an seiner der Luftströmung und/oder dem Außenreflektor (2) und/oder der Lampe (7) zugewandten Seite ein UV-Strahlung reflektierendes Material (10) umfasst,
- wobei der Zentralreflektor (9) die von der Lampe (7) emittierte UV-Strahlung reflektiert, und
- wobei die Längsachse (8) der Lampe (7) schräg zur Längsachse (11) des Zentralreflektors (9) ausgerichtet ist.

2. Luftreiniger nach Anspruch 1,
**dadurch gekennzeichnet, dass** die Längsachse (11) des Zentralreflektors (9) schräg zur Längsachse (6) des Außenreflektors (2) ausgerichtet ist.

3. Luftreiniger nach Anspruch 1 oder 2,
**dadurch gekennzeichnet, dass** der Luftreiniger (1) mindestens zwei Lampen (7) umfasst, dass jede der Lampen (7) als eine UV-Strahlung emittierende stabförmige Lampe (7) ausgebildet ist, die sich jeweils entlang einer Längsachse (8) der jeweiligen Lampe (7) erstreckt, dass jede der Lampen (7) vom Außenreflektor (2) umgeben ist, dass der Außenreflektor (2) und der Zentralreflektor (9) bei eingeschalteten Lampe (7) das von den Lampen (7) emittierte UV-Strahlung reflektieren,
und dass jede der Lampen (7) zwischen Außenreflektor (2) und Zentralreflektor (9) angeordnet ist.

4. Luftreiniger nach Anspruch 3,
**dadurch gekennzeichnet, dass** der Luftreiniger (1) eine sternförmige, lufteinlassseitige Halterung (12) umfasst, wobei jeweils ein Haltesteg (13) das lufteinlassseitige Ende jeweils einer der Lampen (7) mit dem lufteinlassseitigen Ende des Zentralreflektors (9) mechanisch verbindet, und/oder durch eine sternförmige, luftauslassseitige Halterung (16), die Haltestege (17) umfasst, wobei jeweils ein Haltesteg (17) das luftauslassseitige Ende jeweils einer der Lampen (7) mit dem luftauslassseitigen Ende des Zentralreflektors (9) mechanisch verbindet.

5. Luftreiniger nach Anspruch 4,
**dadurch gekennzeichnet, dass** mindestens zwei Haltestege (13, 17) unterschiedlich lang sind.

6. Luftreiniger nach Anspruch 4 oder 5,
**dadurch gekennzeichnet, dass** an einem lufteinlassseitigen und/oder luftauslassseitigen Haltesteg (13, 17) jeweils eine Stromführung zur Versorgung der jeweiligen mit dem Haltesteg (13, 17) verbundenen Lampe (7) mit Strom vorgesehen ist.

7. Luftreiniger nach einem der Ansprüche 4 bis 6,
**dadurch gekennzeichnet, dass** die sternförmige, lufteinlassseitige und/oder luftauslassseitige Halterung (12, 16) über mindestens einen Befestigungssteg (20) mit dem Außenreflektor (2) verbunden ist.

8. Luftreiniger nach einem der Ansprüche 4 bis 7,
**dadurch gekennzeichnet, dass** die in Richtung der Längsachse (6) des Außenreflektors (2) gemessene Dicke der sternförmigen, lufteinlassseitigen und/oder luftauslassseitigen Halterung (12, 16) kleiner ist als die orthogonal zur Längsachse (6) des Außenreflektors (2) gemessene Breite der sternförmigen, lufteinlassseitigen und/oder luftauslassseitigen Halterung (12, 16).

9. Luftreiniger nach einem der Ansprüche 1 bis 8,
**dadurch gekennzeichnet, dass** die Lampe (7) von einem Hüllrohr (21) umgeben ist.

10. Luftreiniger nach Anspruch 9,
**dadurch gekennzeichnet, dass** das die Lampe (7) an ihren Enden jeweils von einer Lampenhalterung (23) gehalten ist, wobei das Hüllrohr (21) an den Lampenhalterungen (23) befestigt ist.

11. Luftreiniger nach Anspruch 9 oder 10,
**dadurch gekennzeichnet, dass** das Hüllrohr (21) die Lampe (7) luftdicht verschließt.

12. Luftreiniger nach einem der Ansprüche 9 bis 11,
**dadurch gekennzeichnet, dass** zwischen Hüllrohr (21) und Lampe (7) ein Freiraum (22) vorgesehen ist.

13. Luftreiniger nach einem der Ansprüche 1 bis 12,
**dadurch gekennzeichnet, dass** der Zentralreflektor (9) mindestens einen Durchlass zur Kühlung des Zentralreflektors (9) mit Luft umfasst.

14. Luftreiniger zur UV-Bestrahlung von insbesondere mit Keimen oder Infektionserregern beladener strömender Luft, mit
- einem bevorzugt zylinderförmig ausgebildeten Außenreflektor (2), der einen Lufteinlass (3) sowie einen Luftauslass (4) umfasst, wobei der Außenreflektor (2) an seiner der Luftströmung zugewandten Innenseite ein UV-Strahlung-reflektierendes Material (5) umfasst, und mehreren, bevorzugt mindestens drei stabförmigen Lampen (7), die eine UV-Strahlung emittieren, wobei der Außenreflektor (2) die von den Lampen (7) emittierte UV-Strahlung reflektiert,
**gekennzeichnet durch**
- einen Zentralreflektor (9), der vom Außenreflektor (2) umgeben ist, wobei der Zentralreflektor (9) an seiner der Luftströmung zugewandten Außenseite ein UV-Strahlung reflektierendes Material (10) umfasst,
- wobei die Lampen (7) zwischen Außenreflektor (2) und Zentralreflektor angeordnet sind,
- und wobei der Zentralreflektor (9) die von den Lampen (7) emittierte UV-Strahlung reflektiert.

15. Luftreiniger nach Anspruch 14,
**dadurch gekennzeichnet, dass** die Lampen (7) in einem Ringraum um den Zentralreflektor (9) angeordnet sind.

## Claims

1. Air purifier for UV irradiation of flowing air, especially air loaded with germs or infectious agents, comprising
- a preferably cylindrical outer reflector (2) extending along a longitudinal axis (6) and having an air inlet (3) and an air outlet (4), the outer reflector (2) having a UV radiation-reflecting material (5) on its inner side facing the air flow,
- a rod-shaped lamp (7) emitting UV radiation and extending along a longitudinal axis (8), the lamp (7) being surrounded by the outer reflector (2), the outer reflector (2) reflecting the UV radiation emitted by the lamp (7),
**characterized by**
- a central reflector (9) extending along a longitudinal axis (11) and surrounded by the outer reflector (2), the central reflector (9) comprising a UV radiation-reflecting material (10) on its side facing the air flow and/or the outer reflector (2) and/or the lamp (7),
- the central reflector (9) reflecting the UV radiation emitted by the lamp (7), and
- the longitudinal axis (8) of the lamp (7) being aligned obliquely to the longitudinal axis (11) of the central reflector (9).

2. Air purifier according to claim 1,
**characterized in that** the longitudinal axis (11) of the central reflector (9) is aligned obliquely to the longitudinal axis (6) of the outer reflector (2).

3. Air purifier according to claim 1 or claim 2,
**characterized in that** the air purifier (1) comprises at least two lamps (7), **in that** each of the lamps (7) is designed as a rod-shaped lamp (7) emitting UV radiation, each of which lamps extends along a longitudinal axis (8) of the relevant lamp (7), **in that** each of the lamps (7) is surrounded by the outer reflector (2), **in that** the outer reflector (2) and the central reflector (9) reflect the UV radiation emitted by the lamps (7) when the lamps (7) are switched on, and **in that** each of the lamps (7) is arranged between the outer reflector (2) and the central reflector (9).

4. Air purifier according to claim 3,
**characterized in that** the air purifier (1) comprises a star-shaped, air inlet-side holder (12), an individual holding strut (13) mechanically connecting the air inlet-side end of each of the lamps (7) to the air inlet-side end of the central reflector (9), and/or by a star-shaped, air outlet-side holder (16) comprising holding struts (17), an individual holding strut (17) mechanically connecting the air outlet-side end of each of the lamps (7) to the air outlet-side end of the central reflector (9).

5. Air purifier according to claim 4,
**characterized in that** at least two holding struts (13, 17) are of different lengths.

6. Air purifier according to claim 4 or claim 5,
**characterized in that** through-wiring is provided on an air inlet-side and/or air outlet-side holding strut (13, 17) respectively for supplying the relevant lamp (7) connected to the holding strut (13, 17) with current.

7. Air purifier according to any of claims 4 to 6,
**characterized in that** the star-shaped, air inlet-side and/or air outlet-side holder (12, 16) is connected to the outer reflector (2) via at least one fixing strut (20).

8. Air purifier according to any of claims 4 to 7,
**characterized in that** the thickness of the star-shaped, air inlet-side and/or air outlet-side holder (12, 16), measured in the direction of the longitudinal axis (6) of the outer reflector (2), is smaller than the width of the star-shaped, air inlet-side and/or air outlet-side holder (12, 16), measured orthogonally to the longitudinal axis (6) of the outer reflector (2).

9. Air purifier according to any of claims 1 to 8,
**characterized in that** the lamp (7) is surrounded by a casing tube (21).

10. Air purifier according to claim 9,
**characterized in that** each end of the lamp (7) is held by an individual lamp holder (23), the casing tube (21) being fixed to the lamp holders (23).

11. Air purifier according to claim 9 or claim 10,
**characterized in that** the casing tube (21) seals the lamp (7) hermetically.

12. Air purifier according to any of claims 9 to 11,
**characterized in that** a free space (22) is provided between the casing tube (21) and the lamp (7).

13. Air purifier according to any of claims 1 to 12,
**characterized in that** the central reflector (9) comprises at least one passage for cooling the central reflector (9) using air.

14. Air purifier for UV irradiation of flowing air, especially air loaded with germs or infectious agents, comprising
- a preferably cylindrical outer reflector (2) having an air inlet (3) and an air outlet (4), the outer reflector (2) having a UV radiation-reflecting material (5) on its inner side facing the air flow, and several, preferably at least three, rod-shaped lamps (7) emitting UV radiation, the outer reflector (2) reflecting the UV radiation emitted by the lamps (7),
**characterized by**
- a central reflector (9) surrounded by the outer reflector (2), the central reflector (9) comprising a UV radiation-reflecting material (10) on its outer side facing the air flow,
- the lamps (7) being arranged between the outer reflector (2) and the central reflector,
- and the central reflector (9) reflecting the UV radiation emitted by the lamps (7).

15. Air purifier according to claim 14,
**characterized in that** the lamps (7) are arranged in an annular space around the central reflector (9).

## Revendications

1. Purificateur d'air permettant l'irradiation UV d'air en circulation chargé en particulier de germes ou d'agents infectieux, comportant
- un réflecteur extérieur (2) réalisé de préférence en forme cylindrique et s'étendant le long d'un axe longitudinal (6), lequel réflecteur extérieur comprend une entrée d'air (3) ainsi qu'une sortie d'air (4), dans lequel le réflecteur extérieur (2) comprend, sur son côté intérieur tourné vers le flux d'air, un matériau (5) réfléchissant un rayonnement UV,
- une lampe (7) en forme de tige émettant un rayonnement UV et s'étendant le long d'un axe longitudinal (8), dans lequel la lampe (7) est entourée du réflecteur extérieur (2), dans lequel le réflecteur extérieur (2) réfléchit le rayonnement UV émis par la lampe (7),
**caractérisé par**
- un réflecteur central (9) s'étendant le long d'un axe longitudinal (11) et étant entouré par le réflecteur extérieur (2), dans lequel le réflecteur central (9) comprend un matériau (10) réfléchissant un rayonnement UV sur son côté tourné vers le flux d'air et/ou vers le réflecteur extérieur (2) et/ou vers la lampe (7),
- dans lequel le réflecteur central (9) réfléchit le rayonnement UV émis par la lampe (7), et
- dans lequel l'axe longitudinal (8) de la lampe (7) est orienté obliquement par rapport à l'axe longitudinal (11) du réflecteur central (9).

2. Purificateur d'air selon la revendication 1,
**caractérisé en ce que** l'axe longitudinal (11) du réflecteur central (9) est orienté obliquement par rapport à l'axe longitudinal (6) du réflecteur extérieur (2).

3. Purificateur d'air selon la revendication 1 ou 2,
**caractérisé en ce que** le purificateur d'air (1) comprend au moins deux lampes (7), **en ce que** chacune des lampes (7) est réalisée sous forme de lampe (7) en forme de tige émettant un rayonnement UV s'étendant respectivement le long d'un axe longitudinal (8) de la lampe (7) respective, **en ce que** chacune des lampes (7) est entourée par le réflecteur extérieur (2), **en ce que** le réflecteur extérieur (2) et le réflecteur central (9) réfléchissent le rayonnement UV émis par les lampes (7) lorsque les lampes (7) sont allumées, **et en ce que** chacune des lampes (7) est disposée entre le réflecteur extérieur (2) et le réflecteur central (9).

4. Purificateur d'air selon la revendication 3,
**caractérisé en ce que** le purificateur d'air (1) comprend un dispositif de maintien (12) en forme d'étoile, côté entrée d'air, dans lequel respectivement une nervure de maintien (13) relie mécaniquement l'extrémité côté entrée d'air de respectivement une des lampes (7) à l'extrémité côté entrée d'air du réflecteur central (9), **et/ou par** un dispositif de maintien (16) en forme d'étoile, côté sortie d'air, comprenant des nervures de maintien (17), dans lequel respectivement une nervure de maintien (17) relie mécaniquement l'extrémité côté sortie d'air de respectivement une des lampes (7) à l'extrémité côté sortie d'air du réflecteur central (9).

5. Purificateur d'air selon la revendication 4,
**caractérisé en ce qu'**au moins deux nervures de maintien (13, 17) sont de longueurs différentes.

6. Purificateur d'air selon la revendication 4 ou 5,
**caractérisé en ce que** respectivement un guidage de courant est prévu sur une nervure de maintien (13, 17) côté entrée d'air et/ou côté sortie d'air pour l'alimentation en courant de la lampe (7) respective reliée à la nervure de maintien (13, 17).

7. Purificateur d'air selon l'une des revendications 4 à 6,
**caractérisé en ce que** le dispositif de maintien (12, 16) en forme d'étoile, côté entrée d'air et/ou côté sortie d'air, est relié au réflecteur extérieur (2) par l'intermédiaire d'au moins une nervure de fixation (20).

8. Purificateur d'air selon l'une des revendications 4 à 7,
**caractérisé en ce que** l'épaisseur du dispositif de maintien (12, 16) en forme d'étoile, côté entrée d'air et/ou côté sortie d'air, mesurée en direction de l'axe longitudinal (6) du réflecteur extérieur (2), est inférieure à la largeur du dispositif de maintien (12, 16) en forme d'étoile, côté entrée d'air et/ou côté sortie d'air, mesurée orthogonalement à l'axe longitudinal (6) du réflecteur extérieur (2).

9. Purificateur d'air selon l'une des revendications 1 à 8,
**caractérisé en ce que** la lampe (7) est entourée d'un tube de gainage (21).

10. Purificateur d'air selon la revendication 9,
**caractérisé en ce que** la lampe (7) est maintenue au niveau de ses extrémités respectivement par un dispositif de maintien pour lampe (23), dans lequel le tube de gainage (21) est fixé aux dispositif de maintien pour lampe (23).

11. Purificateur d'air selon la revendication 9 ou 10,
**caractérisé en ce que** le tube de gainage (21) ferme la lampe (7) de manière étanche à l'air.

12. Purificateur d'air selon l'une des revendications 9 à 11,
**caractérisé en ce qu'**un espace libre (22) est prévu entre le tube de gainage (21) et la lampe (7).

13. Purificateur d'air selon l'une des revendications 1 à 12,
**caractérisé en ce que** le réflecteur central (9) comprend au moins un passage permettant le refroidissement du réflecteur central (9) avec de l'air.

14. Purificateur d'air permettant l'irradiation UV d'air en circulation chargé en particulier de germes ou d'agents infectieux, comportant
- un réflecteur extérieur (2) réalisé de préférence en forme cylindrique et comprenant une entrée d'air (3) ainsi qu'une sortie d'air (4), dans lequel le réflecteur extérieur (2) comprend, sur son côté intérieur tourné vers le flux d'air, un matériau (5) réfléchissant un rayonnement UV, et plusieurs, de préférence au moins trois, lampes (7) en forme de tige émettant un rayonnement UV, dans lequel le réflecteur extérieur (2) réfléchit le rayonnement UV émis par les lampes (7),
**caractérisé par**
- un réflecteur central (9) entouré par le réflecteur extérieur (2), dans lequel le réflecteur central (9) comprend un matériau (10) réfléchissant un rayonnement UV sur son côté extérieur tourné vers le flux d'air,
- dans lequel les lampes (7) sont disposées entre le réflecteur extérieur (2) et le réflecteur central,
- et dans lequel le réflecteur central (9) réfléchit le rayonnement UV émis par les lampes (7).

15. Purificateur d'air selon la revendication 14,
**caractérisé en ce que** les lampes (7) sont disposées dans un espace annulaire autour du réflecteur central (9).
